(19) Europäisches Patentamt
European Patent Office
Office européen des brevets

(11) Veröffentlichungsnummer: **0 274 600**
**B1**

(12) # EUROPÄISCHE PATENTSCHRIFT

(45) Veröffentlichungstag der Patentschrift: **02.08.89**

(51) Int. Cl.[4]: **C07C 43/14**, C07C 41/08

(21) Anmeldenummer: **87116559.3**

(22) Anmeldetag: **10.11.87**

(54) **Verfahren zur Herstellung von Alkoxibuteninen.**

(30) Priorität: **10.01.87 DE 3700605**

(43) Veröffentlichungstag der Anmeldung:
**20.07.88 Patentblatt 88/29**

(45) Bekanntmachung des Hinweises auf die Patenterteilung:
**02.08.89 Patentblatt 89/31**

(84) Benannte Vertragsstaaten:
**AT CH DE LI NL SE**

(56) Entgegenhaltungen:
**DD-A- 127 082**
**DE-B- 1 251 307**

**SOVIET INVENTIONS ILLUSTRATED, Sektion Ch, Woche D 34, 30. September 1981 DERWENT PUBLICATIONS LTD., London**

(73) Patentinhaber: **HÜLS AKTIENGESELLSCHAFT, Patentabteilung / PB 15 - Postfach 13 20, D-4370 Marl 1(DE)**

(72) Erfinder: **Minnerup, Wolfgang, Dr., Griesheimer Strasse 12, D-4370 Marl(DE)**
Erfinder: **Zillmann, Reinhold, Ahornstrasse 23, D-4421 Reken 1(DE)**

**EP 0 274 600 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft ein Verfahren zur Herstellung von Alkoxibuteninen durch Umsetzung von Alkoholen mit Diacetylen in Gegenwart katalytisch wirkender Mengen von Alkalihydroxiden.

$$CH \equiv C - C \equiv CH + ROH \xrightarrow{KOH} CH \equiv C - CH = CHOR$$

Die durch diese Reaktion zugänglichen Alkoxibutenine sind aufgrund ihrer Acetylen- sowie Vinylethergruppen sehr reaktive Verbindungen und stellen wertvolle Zwischenprodukte für organische Synthesen dar. So kann man z. B. Alkohole oder Carbonylverbindungen addieren oder Zyklisierungsreaktionen durchführen.

Die dem erfindungsgemäßen Verfahren zugrundeliegende Reaktion ist seit langem bekannt. So gelangte Herbertz bereits 1952 durch Umsetzung des Diacetylens mit Methanol (über die Zwischenstufe des Diacetylen-Natriumsalzes) zum 1-Methoxi-1-buten-3-in (Chem. Ber. 85 (1952), 475). Einer technischen Verbreitung entsprechender, auf o. g. Grundreaktion basierender Verfahren stand jedoch das mit dem Umgang mit Diacetylen in hohem Maße verbundene Sicherheitsrisiko entgegen. Diacetylen wird aufgrund seiner Neigung zum explosionsartigen Zerfall nahezu ausschließlich zur Herstellung von Laborpräparaten in entsprechend kleinen Mengen benutzt. So findet man in der Literatur die Empfehlung, bei Umsetzungen mit Diacetylen aus Sicherheitsgründen höchstens 10 g Diacetylen einzusetzen (Shostakovskii and Bogdanova, "The Chemistry of Diacetylenes, 1974, Keter Publishing House Jerusalem LTD). Neben dem Diacetylen selbst stellen die bei Umsetzungen mit Diacetylen häufig auftretenden Polymerisatablagerungen eine besondere Gefahrenquelle dar, da sie zum exothermen Zerfall neigen und somit eine potentielle Zündquelle für explosionsartige Zersetzungen darstellen. Außerdem lagern sich diese Polymerisate an Reaktorwandungen und Rohrleitungen ab und erzwingen so in technischen Anlagen infolge des schlechteren Wärmeüberganges bzw. von Verstopfungen häufige Betriebsunterbrechungen. Bei der dem erfindungsgemäßen Verfahren zugrundeliegenden Reaktion tragen außerdem die aus dem Katalysator gebildeteten Alkalisalze in erheblichem Umfang zu den Ablagerungen und Verstopfungen bei. Die jeweils erforderlichen Reinigungsarbeiten sind mit einem nicht unerheblichen Risiko verbunden, da sich die Ablagerungen nur mechanisch entfernen lassen und die hierbei auftretende Beanspruchung u. U. einen Zerfall der Polymerisate initiieren kann.

Es hat nicht an Versuchen gefehlt, trotz der beschriebenen Probleme ein technisch durchführbares Verfahren zur Herstellung von 1-Alkoxi-1-buten-3-inen auszuarbeiten. So beschreibt die DE-AS 12 51 307 ein Verfahren, bei dem versucht wird, durch besondere Ausgestaltung des Reaktors als Röhrenreaktor sowie der Strömungsverhältnisse in dem Reaktor die Bildung von Ablagerungen zu verhindern. Es hat sich jedoch gezeigt, daß allein durch diese Maßnahmen das Problem lediglich gemildert, jedoch nicht verhindert wird. Insbesondere zeigte sich die Unzulänglichkeit dieses Verfahrens bei dem Versuch, für die Alkoxibutenin-Herstellung Alkohole mit mehr als einem C-Atom einzusetzen. Offenbar sind die Löslichkeitsverhältnisse beispielsweise in dem Reaktionsgemisch aus Ethanol, Diacetylen und ethanolischer KOH nochmals ungünstiger als schon in dem Reaktionsgemisch aus Methanol, Diacetylen und methanolischer KOH, so daß die Ausfällung gebildeter Polymerisate sowie von Alkalisalzen begünstigt wird und die Durchführung des Verfahrens wegen Verstopfung des Reaktors bereits nach kurzer Zeit abgebrochen werden muß (s. Beispiele).

Der vorliegenden Erfindung lag daher die Aufgabe zugrunde, ein technisch durchführbares Verfahren für die Herstellung von 1-Alkoxi-1-buten-3-inen durch Umsetzung von Diacetylen mit Alkoholen in Gegenwart katalytisch wirkender Mengen Alkalihydroxid zu entwickeln, bei dem die vorgenannten Beeinträchtigungen durch das Auftreten gefährlicher Polymerisatablagerungen sowie Ablagerungen von Alkalisalzen nicht gegeben sind.

Erfindungsgemäß wird diese Aufgabe entsprechend den Angaben der Patentansprüche dadurch gelöst, daß man dem Reaktionsgemisch bereits vor der Umsetzung höhere Kohlenwasserstoffe zusetzt und diese Mischung der bei Drücken von 4 bis 10 bar und Temperaturen von 80 bis 130 °C durchzuführenden Reaktion zuführt. Es wurde überraschenderweise gefunden, daß nach dem Zusatz der höheren Kohlenwasserstoffe trotz der dadurch bedingten Verdünnung die Reaktion mit tendenziell sogar verbesserter Ausbeute durchgeführt werden kann und Beeinträchtigungen durch Verstopfungen bzw. Ablagerungen völlig unterbleiben.

Das Diacetylen und die Alkalihydroxide setzt man in $C_1$- bis $C_5$-Alkanol-Lösungen ein. Die Alkalihydroxide setzt man im allgemeinen in Mengen von 0,5 bis 5 Gewichtsprozent, bezogen auf die Reaktionsmischung, ein. Bevorzugt setzt man Kaliumhydroxid ein.

Als höhere Kohlenwasserstoffe eignen sich insbesondere solche mit überwiegend aromatischem Charakter, beispielsweise Alkylbenzol, mehrkernige Aromaten, alkylsubstituierte mehrkernige Aromaten oder bei der Herstellung dieser Verbindungen anfallende Rückstandsöle. Bewährt hat sich besonders ein aus der Herstellung von Benzoldialkylat resultierendes Rückstandsöl. Gegebenenfalls können die Alkylreste oder Aromaten mit weiteren funktionellen Gruppen substituiert sein. Diese höheren Kohlenwasserstoffe setzt man bevorzugt in Mengen von 10 bis 200 Gewichtsprozent, bezogen auf das Einsatzgemisch, zu.

Das Verfahren kann beispielsweise, wie im Schema verdeutlicht wird, durchgeführt werden.

Die alkoholische Diacetylenlösung 1 wird in dem Mischgefäß 3 mit der als Katalysator dienenden alkoholischen Alkalihydroxidlösung 2 vermischt. Dieser Reaktionsmischung, die unter einem Druck von ca. 6 bar steht, wird dann der aromatische Kohlenwasserstoff 4 zugesetzt, bevor die gesamte Mischung in dem mit Niederdruckdampf beheizten Aufheizer 5 auf die erforderliche Reaktionstemperatur gebracht wird. Anschließend wird das Gemisch durch den mit Niederdruckdampf beheizten Reaktor 6 gefahren, bevor das Gemisch schließlich in den Dünnschichtverdampfer 8 entspannt wird. Im Dünnschichtverdampfer werden die niedrig siedenden Bestandteile 9 (im wesentlichen Alkoxibutenin und Alkohol) von den höhersiedenden Bestandteilen 10 (aromatische Kohlenwasserstoffe, Kaliumalkoholat, Polymerisate) getrennt. Das Alkoxibutenin wird schließlich destillativ weiter aufkonzentriert, wobei in der Regel aus Sicherheitsgründen eine ca. 50 %ige alkoholische Lösung hergestellt wird.

Vergleichsbeispiel A (Stand der Technik)

480 l/h einer 7 %igen ethanolischen Diacetylenlösung werden mit 45 kg/h einer 20 %igen ethanolischen Kaliumhydroxidlösung vermischt. Im Vorwärmer wird die Mischung auf ca. 100 °C aufgeheizt, im anschließenden Reaktor findet bei ca. 100 °C und 6 bar die Umsetzung des Diacetylens mit Ethanol zum Ethoxibutenin statt. Nach dem Austritt aus dem Reaktor wird der Reaktionsmischung ca. 200 l/h eines aromatischen Rückstandsöles zugesetzt. Nach Entspannung des Gemisches in den Dünnschichtverdampfer geht bei ca. 60 °C und 200 mbar ein Gemisch aus im wesentlichen Ethanol und Ethoxibutenin über, welches sich schließlich durch Abtreiben von Ethanol zu 91 kg/h einer 50 %igen ethanolischen Ethoxibuteninlösung aufkonzentrieren läßt. Auf das eingesetzte Diacetylen bezogen entspricht die so erhaltene Ethoxibuteninmenge einer Ausbeute von 88,2 %.

Das Verfahren läßt sich auf die hier beschriebene Art allerdings nur eine kurze Zeit durchführen, weil bereits nach drei bis vier Stunden der Reaktor mit Ablagerungen verstopft ist und die Anlage abgestellt und gereinigt werden muß.

Beispiel 1 (erfindungsgemäß)

Wie im Vergleichsversuch A werden 480 l/h einer 7 %igen ethanolischen Diacetylenlösung mit 45 kg/h einer 20 %igen ethanolischen Kaliumhydroxidlösung vermischt und unter den gleichen Reaktionsbedingungen wie in Beispiel 1 miteinander umgesetzt. Anders als in Beispiel 1 jedoch werden dem Reaktionsgemisch bereits vor Eintritt in den Vorwärmer 200 l/h eines aromatischen Rückstandsöles zugemischt. Nach Austritt aus dem Reaktor wird das Gemisch in den Dünnschichtverdampfer entspannt, aus den bei 60 °C und 200 mbar übergehenden Komponenten lassen sich durch destillative Aufkonzentrierung 94 kg/h einer 50 %igen ethanolischen Ethoxibuteninlösung gewinnen. Auf das eingesetzte Diacetylen bezogen entspricht die so erhaltene Ethoxibuteninmenge einer Ausbeute von 91,1 %.

Das Verfahren läßt sich ohne Betriebsunterbrechung mehr als 3 Monate lang betreiben. Auch nach dieser Zeitspanne werden keine Anzeichen von Ablagerungen im Reaktor festgestellt.

## Patentansprüche

1. Verfahren zur Herstellung von Alkoxibuteninen durch Umsetzung einer Lösung von Diacetylen in $C_1$- bis $C_5$-Alkanolen in Gegenwart von Alkalihydroxiden bei Temperaturen von 80 bis 130 °C und Drücken von 4 bis 10 bar, dadurch gekennzeichnet, daß man die Umsetzung in Gegenwart von höheren aromatischen Kohlenwasserstoffen durchführt.

2. Verfahren nach Anspruch 1, dadurch gekennzeichnet, daß man die höheren Kohlenwasserstoffe in Mengen von 10 bis 200 Gewichtsprozent, bezogen auf das Einsatzgemisch, zugibt.

3. Verfahren nach einem der Ansprüche 1 oder 2, dadurch gekennzeichnet, daß man als höhere Kohlenwasserstoffe Alkylbenzol, mehrkernige Aromaten, alkylsubstituierte mehrkernige Aromaten oder bei Herstellung dieser Verbindungen anfallende Rückstandsöle, vorzugsweise ein bei der Herstellung von Benzoldialkylat anfallendes Rückstandsöl, einsetzt.

## Claims

1. A process for the production of alkoxybutenines by reacting a solution of diacetylene in $C_1$ to $C_5$ alcohols in the presence of alkali hydroxide at temperatures of 80 to 130°C and pressures of 4 to 10 bars, characterized in that the reaction is performed in the presence of higher aromatic hydrocarbons.

2. A process according to Claim 1, characterized in that the higher hydrocarbon is added in amounts of 10 to 200 percent by weight, based on the starting mixture.

3. A process according to Claim 1 or Claim 2, characterized in that the higher hydrocarbon is an alkylbenzene, a polycyclic aromatic compound, an alkyl substituted polycyclic aromatic compound, or a residual oil produced in the production of these compounds, preferably a residual oil produced in the production of dialkylated benzene.

## Revendications

1. Procédé d'obtention d'alcoxybutenynes par transformation d'une solution d'un diacétylène dans des alcanols en $C_1$–$C_5$ en présence d'hydroxydes alcalins à des températures de 80–130°C et des pression de 4 à 10 bars, caractérisé en ce que l'on effectue la transformation en présence d'hydrocarbures aromatiques de poids moléculaires plus élevé.

2. Procédé selon la revendication 1, caractérisé en ce que l'on ajoute les hydrocarbures de poids moléculaire plus élevé en quantités de 10 à 200% en poids rapporté au mélange mis en œuvre.

3. Procédé selon l'une des revendications 1 ou 2, caractérisé en ce qu'on met en œuvre comme hydrocarbures à poids moléculaire plus élevé, un alcoylbenzène, des aromatiques à plusieurs cycles, des aromatiques polycycliques substitués par un alcoyl ou des huiles résiduelles qui se forment au cours de la production de ces composés, de préférence une huile résiduelle qui se forme au cours de la production de benzène dialcoylate.

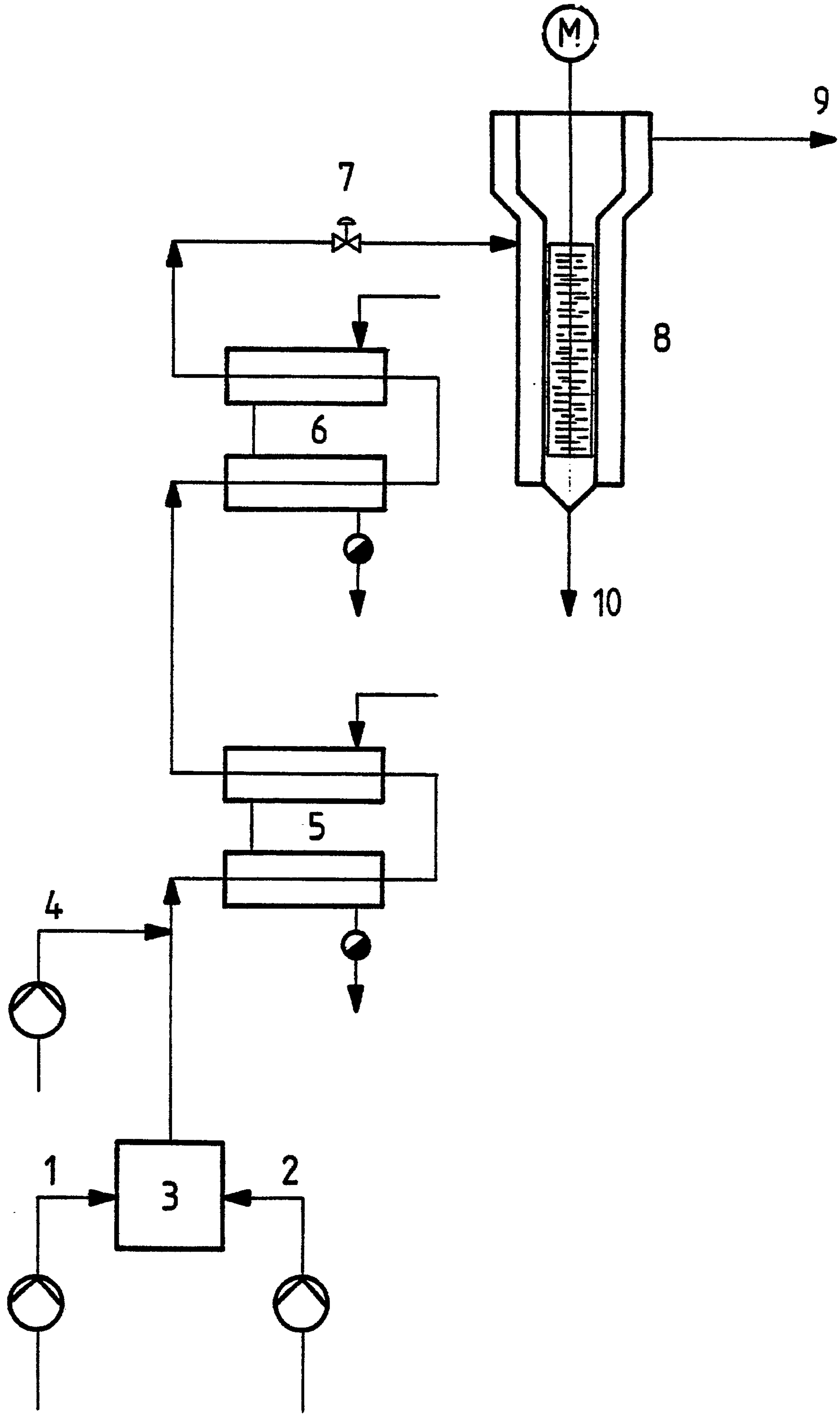
M
9
7
8
6
10
5
4
1
3
2